# EUROPÄISCHE PATENTANMELDUNG

(11) **EP 2 835 077 A1**
(43) Veröffentlichungstag der Anmeldung: **11.02.2015**
(21) Anmeldenummer: 14180324.7
(22) Anmeldetag: 08.08.2014
(51) Int. Cl.: A47G 9/02, A01K 13/00

(54) **Wohlfühl- und/oder Wärmedecke**

(30) Priorität: 09.08.2013 DE 102013215780
(71) Anmelder: IBENA Textilwerke GmbH, 46395 Bocholt (DE)
(72) Erfinder: Smeulders, Britta, 7131 WD Lichtenvoorde (NL)
(74) Vertreter: Hoffmann Eitle

(57) **Zusammenfassung**

Eine Wohlfühl- und/oder Wärmedecke weist autonome, optisch leitfähige Fasern auf, die mit einem Anteil von 40 g/m² oder weniger, bevorzugt 37 g/m² oder weniger und insbesondere etwa 35 g/m² vorliegen und/oder die einen Gewichtsanteil von weniger als 15 %, bevorzugt weniger als 10 % und insbesondere 8 % oder weniger ausmachen.

## Beschreibung

### TECHNISCHES GEBIET

Die Erfindung betrifft eine Wohlfühl- und/oder Wärmedecke, die nachfolgend der Einfachheit halber lediglich als Decke bezeichnet wird, und dem allgemeinen Wohlbefinden dient, wobei für die erfindungsgemäße Decke festgestellt wurde, dass sie der bioenergetischen Stimulanz dient und eine Heilwirkung erzeugen kann.

### STAND DER TECHNIK

Decken sind sowohl für Menschen als auch für Tiere, wie zum Beispiel Pferde bekannt, um den Körper des Trägers warm zu halten. Darüber hinaus sind verschiedenste Ausführungen von Heildecken bekannt.

Beispielsweise geht aus der CN 202314978 U eine Decke hervor, die Glasfasern als Lichtleiter aufweist, in die blaues Licht eingespeist werden kann, um dadurch heilend zu wirken. Einen ähnlichen Gegenstand betrifft die KR 20030069497 A.

Darüber hinaus sind Decken bekannt, die beispielsweise zur Gewährleistung einer besonderen Feuerfestigkeit einen hohen Glasfaseranteil aufweisen.

### DARSTELLUNG DER ERFINDUNG

Der Erfindung liegt die Aufgabe zugrunde eine Wohlfühl- und/oder Wärmedecke zu schaffen, die eine verbesserte Wirkung aufweist.

Die Lösung dieser Aufgabe erfolgt durch die im Anspruch 1 beschriebene Decke.

Demzufolge weist diese autonome, optisch leitfähige Fasern mit einem Gewichtsanteil von weniger als 15 %, bevorzugt weniger als 10 % und insbesondere 8 % oder weniger, und besonders bevorzugt etwa 7 % auf. Alternativ oder ergänzend liegt der Anteil der optisch leitfähigen Fasern bei 40 g/m² oder weniger, bevorzugt 37 g/m² oder weniger und insbesondere bei etwa 35 g/m². Unter "autonomen", optisch leitfähigen Fasern wird verstanden, dass diese keinen Anschluss oder dergleichen für die Einspeisung von Licht und/oder elektrischer Energie aufweisen. Vielmehr sind erfindungsgemäß, wie nachfolgend genauer beschrieben, die optisch leitfähigen Fasern in dem für die Decke verwendeten Garn vorgesehen, und es hat sich herausgestellt, dass sich hierdurch eine heilende Wirkung erzielen lässt. Für das Material der optisch leitfähigen Fasern wird Glas bevorzugt. Diese können jedoch auch aus Quarz (SiO2), anderen Mineralien, Acrylat, aus dem beispielsweise Kunststoffgläser für Brillen gefertigt sind, oder einer Kombination der genannten Materialien ausgeführt sein. Der Einfachheit halber werden die optisch leitfähigen Fasern nachfolgend als Glasfasern bezeichnet.

Bevorzugte Weiterbildungen der erfindungsgemäßen Decke sind in den weiteren Ansprüchen beschreiben.

Bei ersten Versuchen wurden besonders gute Erfahrungen mit Glasfasern gemacht, die als Filamente vorliegen.

Für den Einsatz der Glasfasern wird ferner bevorzugt, dass diese als Seele in einem sogenannten Coregarn verwendet werden, das mit anderen Worten an seiner Außenseite übliche, für Decken verwendete Fasern, beispielsweise aus Baumwolle, aufweist. Hierdurch ergibt sich der Vorteil, dass die Glasfasern für den Benutzer nicht in Erscheinung treten, sondern die Optik und Haptik einer "gewöhnlichen" Decke entsteht. Insbesondere handelt es sich bei der erfindungsgemäßen Decke somit um ein weitgehend "normales" textiles Flächengebilde, das insbesondere auch waschbar ist. Hierbei sind die spröden, optisch leitfähigen Fasern, die im Fall von Glaserfasern leicht brechen und deshalb zu Juckreiz führen können, in vorteilhafter Weise im Inneren der Decke "versteckt". Alternativ oder ergänzend können die optisch leitfähigen Fasern in einem Umwindegarn vorgesehen sein. Weiter alternativ oder ergänzend können die optisch leitfähigen Fasern silkonisiert und/oder in sonstiger geeigneter Weise beschichtet sein, um die Anmutung einer gewöhnlichen und insbesondere waschbaren Wohlfühldecke zu erhalten.

Insbesondere wird derzeit bevorzugt, dass das die Glasfasern aufweisende Garn als Kettgarn eingesetzt wird. Dadurch, dass die Schussgarne beispielsweise mit Ober- und Unterschuss ausgeführt sein können, wird das die optisch leitfähigen Fasern aufweisende Kettgarn abgedeckt und im Inneren der Decke gehalten.

Für die Heilwirkung der erfindungsgemäßen Decke bietet es ferner Vorteile, wenn die Glasfasern darin gespeicherte Photonen, insbesondere Bio-Photonen aufweisen.

Die Wirkung der erfindungsgemäßen Decke ist ferner besonders umfangreich, wenn die Glasfasern geeignet sind, Photonen, insbesondere Bio-Photonen abzugeben, was über einen längeren Zeitraum geschieht. Somit wird, wenn auch in kaum messbarem Umfang sichtbares Licht, das heißt Licht mit einer Wellenlänge im Bereich von 200 bis 800 Nanometer ausgesendet.

Während an die Gestalt einer für den Menschen geeigneten Decke keine besonderen Anforderungen gestellt sind, wird in einer Ausführungsform bevorzugt, dass diese als Pferde-, Hunde- oder Katzendecke ausgeführt und entsprechend gestaltet und/oder konfektioniert ist. Darüber hinaus kann die erfindungsgemäße Decke für jegliche weiteren Tiere, insbesondere Haustiere angepasst sein.

## Patentansprüche

1. Wohlfühl- und/oder Wärmedecke mit autonomen, optisch leitfähigen Fasern, die mit einem Anteil von 40 g/m² oder weniger, bevorzugt 37 g/m² oder weniger und insbesondere etwa 35 g/m² vorliegen und/oder die einen Gewichtsanteil von weniger als 15 %, bevorzugt weniger als 10 % und insbesondere 8 % oder weniger ausmachen.

2. Decke nach Anspruch 1, **dadurch gekennzeichnet, dass** die optisch leitfähigen Fasern als Filamente vorliegen.

3. Decke nach Anspruch 1 oder 2, **dadurch gekennzeichnet, dass** die optisch leitfähigen Fasern als Seele eines Coregarns vorgesehen sind.

4. Decke nach einem der vorangehenden Ansprüche, **dadurch gekennzeichnet, dass** die optisch leitfähigen Fasern im Kettgarn vorgesehen sind.

5. Decke nach einem der vorangehenden Ansprüche, **dadurch gekennzeichnet, dass** Photonen, bevorzugt Bio-Photonen in den optisch leitfähigen Fasern gespeichert sind.

6. Decke nach einem der vorangehenden Ansprüche, **dadurch gekennzeichnet, dass** die optisch leitfähigen Fasern geeignet sind, Photonen, bevorzugt Bio-Photonen abzugeben.

7. Decke nach einem der vorangehenden Ansprüche, **dadurch gekennzeichnet, dass** diese als Pferde-, Hunde- oder Katzendecke ausgeführt ist.
